# EUROPEAN PATENT SPECIFICATION

(11) **EP 2 436 316 B1**
(45) Date of publication and mention of the grant of the patent: **21.03.2018**
(21) Application number: 11183317.4
(22) Date of filing: 29.09.2011
(51) Int. Cl.: A61B 17/04

(54) **Suture anchor with suture management**
Nahtanker mit Nahtverwaltung
Ancrage de suture avec gestion des sutures

(30) Priority: 30.09.2010 US 388028 P
(43) Date of publication of application: 04.04.2012
(73) Proprietor: DePuy Mitek, Inc., Raynham, MA 02767 (US)
(72) Inventor: DiMatteo, Kristian, Raynham, Massachusetts 02767 (US); Whittaker, Gregory R., Raynham, Massachusetts 02767 (US); Cauldwell, Nathan, Raynham, Massachusetts 02767 (US)
(74) Representative: Tunstall, Christopher Stephen

(56) References cited:
- WO-A1-02/11630
- WO-A2-02/21998

## Description

### Background

This invention relates to suture anchors for attaching suture to bone, and more specifically to such suture anchors which employ a suture management feature.

It is known to use suture anchors for attaching soft tissues such as ligaments or tendons to adjacent bone. Threaded suture anchors employ external threads and are rotated into the bone, usually into a pre-drilled bone hole, to affix the suture anchor to the bone. They will typically employ an eyelet or other feature for affixing one or more sutures to the anchor. Managing the sutures extending from the suture anchor can be a challenge.

WO 02/11630 describes a bone anchoring system including a bone screw defining a self-tapping, self-boring tip and a central bore, and a mechanical insert having a distal end received in the central bore and mechanically held by the bone screw. The insert has a proximal end defining an attachment structure for securing to suture thread, tissue or other prosthetic device, and the bone screw includes at least one opening for receipt of new bone growth when the bone screw is screwed in place in bone. In particular, the bone screw includes a shank defining screw threads, and the shank further defines transverse through-holes for receipt of new bone growth. The central bore terminates in the proximal end of the bone screw so that the distal end of the bone screw (except for the through-holes) is essentially solid. The distal end of the insert threads into the bone screw, or is snap fitted therein so as to be rotatable with respect to the bone screw.

WO 02/21998 describes a suture anchor including a body extending along a longitudinal axis and having an external threaded portion and a bore extending from the proximal end towards the distal end. Two pairs of slots extend laterally outwardly from the bore. An eyelet is received within the first and the second pairs of slots and has an opening for receiving a suture, and a spring biases the eyelet into the bore. The spring is an axial tension spring within the bore attached to the body and the eyelet. The bore includes a socket and the spring includes a mooring rotatably secured in the socket.

### Summary of the Invention

A suture anchor according to the present invention comprises an anchor body having a longitudinal axis and bone engaging external threads oriented for rotation about the longitudinal axis. A suture attachment on the anchor body is rotational with respect to the body about the longitudinal axis whereby to help reduce twisting of one or more sutures which may be attached to the suture attachment as the anchor body is threaded into a bone. An axial bore enters the body from a proximal end thereof with the suture attachment located on the body such that one or more sutures attached thereto can extend out of the axial bore at the body proximal end. The suture attachment comprises a disc having a plurality of axial holes therethrough for receiving one or more loops of suture therethrough, or a cylinder bearing a plurality of suture eyelets that pass laterally through the cylinder and are spaced apart both axially and rotationally.

Preferably, the body and suture attachment are provided sterile and packaged in a bacteria proof enclosure. One or more sutures can be attached to the suture attachment.

Preferably, a loop of suture is affixed to the suture attachment, the loop having ends extending outwardly from the suture anchor body and suture attachment. Also preferably, a plurality of suture loops of suture are affixed to the suture attachment, each loop having ends extending outwardly from the suture anchor body and suture attachment.

Preferably, the body comprises a first abutment surface within the axial bore and the suture attachment comprises an opposing second abutment surface engaged with the first abutment surface such that the suture attachment is rotatable in the axial bore against the first abutment surface with the engagement between the first abutment surface and the second abutment surface preventing the suture attachment from moving proximally out of the axial bore. Preferably, the first abutment surface and second abutment surface are annular.

In one aspect of the invention, the suture attachment comprises a proximal cylindrical portion having one or more radially extending bosses thereon, the cylindrical portion being received within the axial bore with the one or more bosses adjacent a wall of the anchor body forming the axial bore such that suture looped about the one or more bosses is retained by the one or more bosses within the body between the wall and cylindrical portion.

### Brief Description of the Drawings

FIG. 1 is an exploded perspective view of a suture anchor according to the present invention;FIG. 2 is a perspective view of a suture receiver of the suture anchor of FIG. 1;FIG. 3 is a perspective view of the suture receiver of FIG. 2 with an alternative method for engaging multiple suture loops;FIG. 4 is a cut-away view of the suture anchor of FIG. 1 being implanted into a bone;
FIG. 5 is an exploded perspective view of another suture anchor which does not form part of the invention;
FIG. 6 is an exploded perspective view of a further embodiment of a suture anchor according to the present invention;
FIG. 7A is a perspective view of another suture anchor which does not form part of the invention;
FIG. 7B is an exploded perspective view of the distal portion of the suture anchor of FIG. 7A; and FIG. 7C is a cut-away view of the distal portion of the suture anchor of FIG. 7A.

### Detailed Description

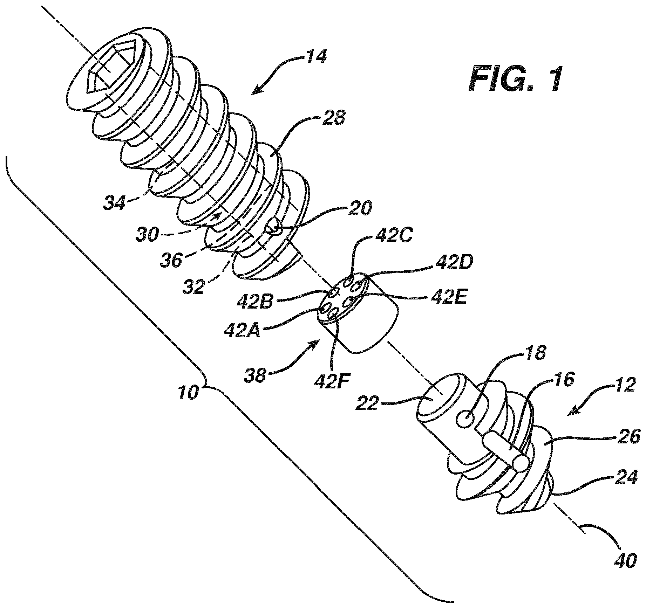

FIG. 1 illustrates a suture anchor 10 according to the present invention. Suture anchor 10 comprises a distal body portion 12 which connects to a proximal body portion 14 both of which are held together by a pin 16 through respective cross-bores 18 and 20 in the distal body portion 12 and the proximal body portion 14. The distal body portion 12 is generally cylindrical in shape having a proximal end 22 through which the cross-bore 18 passes and a distal end 24 which tapers inwardly for easier insertion of the suture anchor 10 into a bone hole (not shown in FIG. 1). An external screw thread 26 encircles the distal body portion 12.

The proximal body portion 14 is also generally cylindrical in shape and bears an external screw thread 28 which mates with the thread 26 to form a continuous threading about the suture anchor 10. An axial-bore 30 passes through the proximal body portion 14. A distal portion 32 thereof, has a larger diameter than a proximal portion 34 thereby creating an internal annual abutment 36. The proximal portion 34 has a hexagonal cross-section to accommodate a driving tool (not shown in FIG. 1). One with ordinary skill in the art would appreciate that alternative tool/anchor engagements may be employed.

The bore-distal portion 32 has a circular cross section and is sized to closely accommodate the body distal portion proximal end 22. A suture receiver 38 fits within the axial-bore distal portion 32 between the abutment 36 and the distal body portion proximal end 22. It has a circular cross-section and is sized to rotate freely about a longitudinal axis 40 of the suture anchor 10. Six axial suture bores 42A-F pass through the suture receiver 38.

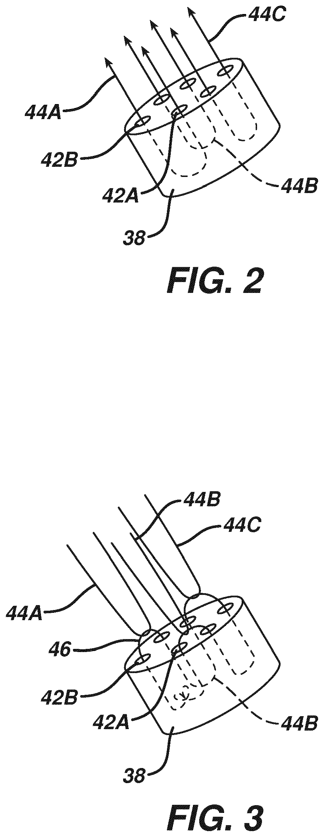

Turning also now to FIG. 2, the suture bores 42A-F accommodates three suture loops 44A-C. Alternatively, as shown in FIG. 3, a suture strand 46 can be woven through the suture bores 42A-F such that the suture loops 44A-C can be threaded through portions of the suture strand 46 extending above the suture receiver 38. Ends 48 of the each of the suture loops 44A-C extend proximally out of the suture anchor 10 through the axial bore 30. The suture ends 48 can then be attached to soft tissue as is understood by one of skill in the art for attaching that soft tissue to a bone. For instance, in a rotator cuff repair, each of the suture loops 44A-C may extend through the rotator cuff tendon at different locations to effect a repair.

The design of the suture anchor 10 provides advantages both in manufacturability and in performance. Having the suture loops 44A-C attach internal of the suture anchor and extending out through the axial bore 30, provides a preferred angle of exit toward soft tissue versus a proximal attachment point. With the separate distal body portion 12 and proximal body portion 14 attaching the suture loops 44A-C deep within the suture anchor 10 becomes easier during manufacturing. When inserting a conventional threaded anchor, any suture loops extending therefrom tend to twist due to the twisting of the anchor in its insertion. The present suture receiver 38 is free to rotate within the axial bore 30 thus reducing twisting of the suture loops 44A-C as the suture anchor 10 is inserted.

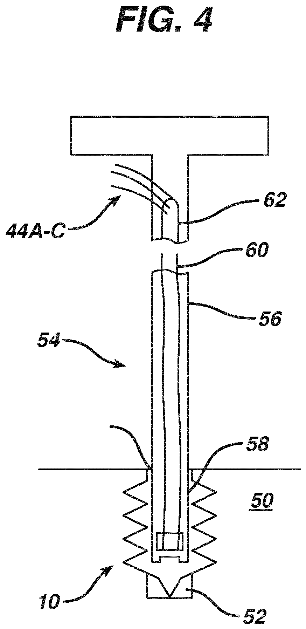

Turning also now to FIG. 4, preferably, the suture anchor 10 is inserted into a bone 50 having a pre-drilled hole 52. A driver 54 having an elongated body 56 and an external hex shape at its distal tip 58, mates with the hex shaped axial bore proximal portion 34. The suture loops 44A-C extend proximally out of the suture anchor 10 through a canulation 60 through the driver distal tip 58 and body 56. In one preferred embodiment, the canulation 60 is open 62 along one side much that after the suture anchor is driven into the bone hole 52 by the driver 54, the driver 54 can be removed from the suture anchor 10 with the suture loops 44A-C disengaging from the driver 54 through the canulation opening 62.

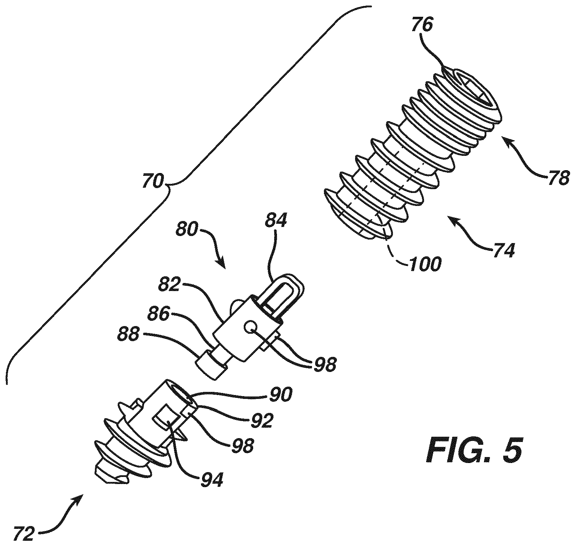

FIG. 5 illustrates an alternative suture anchor 70 comprising a distal threaded body portion 72 and proximal threaded body portion 74. The suture anchor 70 is similar in most respects to the suture anchor 10. However, the proximal body portion 74 carries an additional thread start 76 at its proximal portion 78 thereby effectively increasing thread pitch at the proximal portion 78 and thus enhancing purchase within the hard cortical bone (not shown in FIG. 5) with which this portion typically engages. Also, the anchor 70 employs a somewhat different suture receiver 80.

The suture receiver 80 comprises a central cylindrical body 82 having a proximally extending suture eyelet 84. A distally extending post 86 terminates in an annular flange 88. The anchor distal body portion 72 has an axial bore 90 having a distal body portion 92 sized to accommodate the flange 88 and a narrower proximal portion 94 sized to accommodate the post 86 with the diameter differences creating an internal annular abutment 96 against which the flange 88 bears preventing its proximal movement out of the bore 90. A lateral cut out 98 is provided in the distal body portion 72 to allow the flange 88 and post 86 to be inserted laterally into distal bore 90. This arrangement allows the suture receiver 80 to rotate freely within the distal bore (90).

Several bosses 98 extend outwardly radially from the suture receiver body 82 and fit closely, yet with rotation, within an axial bore 100 in the proximal body portion 74. Suture loops (not shown in FIG.5) can be passed through the eyelet 84 and also, if desired, around each of the bosses 98, and then proximally out of the anchor through the bore 100, the loops about the bosses being held by the bosses 98 and not being able to slip between the bosses 98 and the body 74 due to the close fit of the bosses 98 within the bore 100.

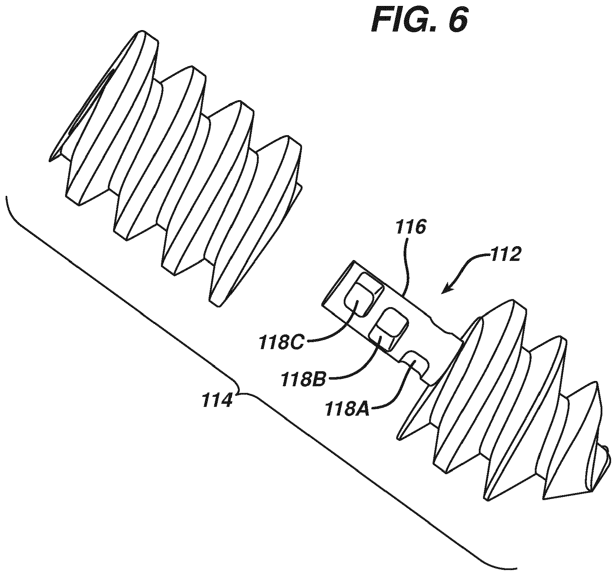

FIG. 6 illustrates a further embodiment of a suture anchor 110 according to the invention similar to that shown in FIG. 5. A suture receiver 112 is rotatably received within a distal body portion 114 as in the previous embodiment however its proximal end 116 is structured differently. It is cylindrical and bears three suture eyelets 118 A-C which are spaced apart both axially and rotationally, with the middle eyelet 118 B passing laterally through the proximal end 116 at 45 degrees with respect to the top eyelet 118 A and bottom eyelet 118 C and with the top and bottom eyelets 118 A and 118 C passing laterally through the proximal end and being oriented normal to each other. The placement and orientation of the eyelets 118 A-C assists in suture management, both in separation and in identification of individual loops.

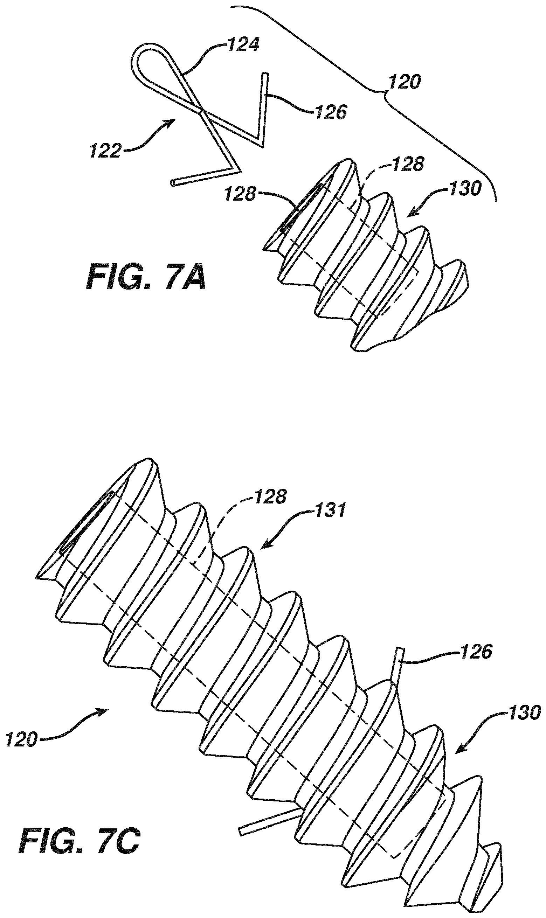

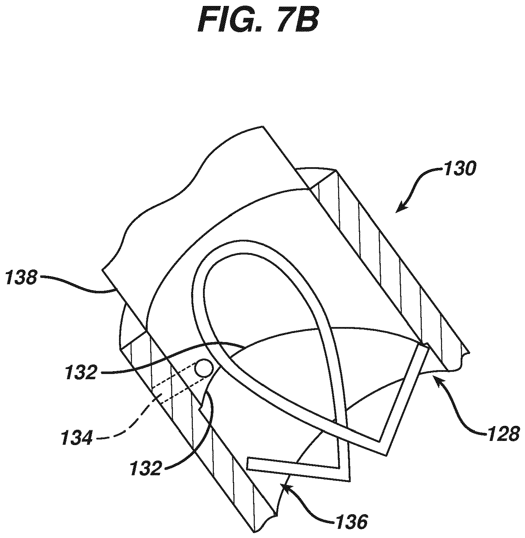

FIGS. 7A-C. illustrate a further suture anchor 120 having an alternative suture receiver 122 in the form of a bent loop 124 of spring metal having proximally extending ends 126. It is received within an axial bore 128 of a distal body portion 130 of the anchor 120 (which attaches to a proximal body portion 131). One or more lengths of suture (not shown in FIGS. 7A-C) can attach to the loop 124. Proximal force therefrom draws the ends 126 up along a camming ledge 132 within the bore 128 toward angled side ports 134 from the bore 128 through the distal body portion 130, allowing the ends 126 to protrude from the distal body portion 130 (see FIG. 7 C) and provide additional fixation within a bone (not shown in FIGS. 7A-C). While the anchor 120 is being inserted a distal end 136 of a cannulated insertion tool 138 keep the ends 126 within the bore 128 and distal of the ports 134 while allowing rotation of the suture receiver 122 within the bore 128.

## Claims

1. A suture anchor (10; 110) comprising:
an anchor body (12, 14; 114) comprising a longitudinal axis, bone engaging external threads (26, 28) oriented for rotation about the longitudinal axis and an axial bore (30) entering the body from a proximal end (14) thereof; and
a suture attachment (38; 112) located on the anchor body such that one or more sutures (44A-C) attached thereto can extend out of the axial bore at the body proximal end, the suture attachment being rotational with respect to the body about the longitudinal axis whereby to help reduce twisting of one or more sutures which may be attached to the suture attachment as the anchor body is threaded into a bone;
**characterized in that** the suture attachment comprises a disc (38) having a plurality of axial holes (42A-F) through it for receiving one or more loops of suture (44A-C), or a cylinder (116) bearing a plurality of suture eyelets (118A-C) that pass laterally through the cylinder and are spaced apart both axially and rotationally.

2. The suture anchor of claim 1 wherein the body and suture attachment are sterile and packaged in a bacteria proof enclosure.

3. The suture anchor of claim 1 wherein at least one suture (44A-C) is attached to the suture attachment.

4. The suture anchor of claim 3 wherein the at least one suture comprises a loop of suture affixed to the suture attachment, the loop having ends (48) extending outwardly from the suture anchor body and suture attachment.

5. The suture anchor of claim 3 wherein the at least one suture comprises a plurality of suture loops (44A-C) of suture affixed to the suture attachment, each loop having ends extending outwardly from the suture anchor body and suture attachment.

6. The suture anchor of claim 1 wherein the body comprises a first abutment surface (36; 96) within the axial bore and wherein the suture attachment comprises an opposing second abutment surface (88) engaged with the first abutment surface such that the suture attachment is rotatable in the axial bore against the first abutment surface and the engagement between the first abutment surface and the second abutment surface prevents the suture attachment from moving proximally out of the axial bore.

7. The suture anchor of claim 6 wherein the first abutment surface and second abutment surface are annular.

## Patentansprüche

1. Nahtanker (10; 110), umfassend:
einen Ankerkörper (12, 14; 114), umfassend eine Längsachse, Knochen in Eingriff nehmende Außengewinde (26, 28), die zur Drehung um die Längsachse orientiert sind, und eine axiale Bohrung (30), die in den Körper von einem proximalen Ende (14) davon eintritt; und
eine Nahtmaterialbefestigung (38; 112), die derart auf dem Ankerkörper angeordnet ist, dass ein oder mehrere daran befestigte Nahtmaterialien (44A-C) sich aus der axialen Bohrung am proximalen Ende des Körpers erstrecken können, wobei die Nahtmaterialbefestigung bezüglich des Körpers um die Längsachse drehbar ist, um dadurch dazu beizutragen, dass Verdrehen von einem oder mehreren Nahtmaterialien, die an der Nahtmaterialbefestigung befestigt sein können, reduziert wird, wenn der Ankerkörper in einen Knochen eingeführt wird;
**dadurch gekennzeichnet, dass** die Nahtmaterialbefestigung eine Scheibe (38) mit einer Vielzahl von axialen Löchern (42A-F) durch sie hindurch zur Aufnahme von ein oder mehreren Nahtmaterialschleifen (44A-C) oder einen Zylinder (116), der eine Vielzahl von Nahtösen (118A-C) trägt, die seitlich durch den Zylinder verlaufen und sowohl axial als auch drehend voneinander beabstandet sind, umfasst.

2. Nahtanker nach Anspruch 1, wobei der Körper und die Nahtmaterialbefestigung steril und in einer bakteriendichten Umhüllung verpackt sind.

3. Nahtanker nach Anspruch 1, wobei mindestens ein Nahtmaterial (44A-C) an der Nahtmaterialbefestigung befestigt ist.

4. Nahtanker nach Anspruch 3, wobei das mindestens eine Nahtmaterial eine Nahtmaterialschleife umfasst, die an der Nahtmaterialbefestigung befestigt ist, wobei die Schleife Enden (48) aufweist, die sich vom Nahtankerkörper und der Nahtmaterialbefestigung nach außen erstrecken.

5. Nahtanker nach Anspruch 3, wobei das mindestens eine Nahtmaterial eine Vielzahl von Nahtmaterialschleifen (44A-C) des Nahtmaterials umfasst, die an der Nahtmaterialbefestigung befestigt sind, wobei jede Schleife Enden aufweist, die sich vom Nahtankerkörper und der Nahtmaterialbefestigung nach außen erstrecken.

6. Nahtanker nach Anspruch 1, wobei der Körper eine erste Anlagefläche (36; 96) in der axialen Bohrung umfasst und wobei die Nahtmaterialbefestigung eine gegenüberliegende zweite Anlagefläche (88) umfasst, die mit der ersten Anlagefläche in Eingriff ist, derart, dass die Nahtmaterialbefestigung in der axialen Bohrung gegen die erste Anlagefläche drehbar ist und der Eingriff zwischen der ersten Anlagefläche und der zweiten Anlagefläche verhindert, dass sich die Nahtmaterialbefestigung proximal aus der axialen Bohrung bewegt.

7. Nahtanker nach Anspruch 6, wobei die erste Anlagefläche und die zweite Anlagefläche ringförmig sind.

## Revendications

1. Ancrage de suture (10 ; 110) comprenant :
un corps d'ancrage (12, 14 ; 114) comprenant un axe longitudinal, des filets externes entrant en prise avec l'os (26, 28) orienté pour une rotation autour de l'axe longitudinal et un alésage axial (30) entrant dans le corps à partir d'une extrémité proximale (14) de celui-ci ; et
une fixation de suture (38 ; 112) située sur le corps d'ancrage de telle sorte qu'une ou plusieurs sutures (44A-C) fixées à celui-ci peuvent s'étendre hors de l'alésage axial au niveau de l'extrémité proximale du corps, la fixation de suture étant rotative par rapport au corps autour de l'axe longitudinal, aidant ainsi à réduire la torsion d'une ou de plusieurs sutures qui peuvent être fixées à la fixation de suture lorsque le corps d'ancrage est vissé dans un os ;
**caractérisé en ce que** la fixation de suture comprend un disque (38) ayant une pluralité de trous axiaux (42A-F) à travers celui-ci pour recevoir une ou plusieurs boucles de suture (44A-C), ou un cylindre (116) portant une pluralité d'oeillets de suture (118A-C) qui passent latéralement à travers le cylindre et sont espacés les uns des autres axialement et en rotation.

2. Ancrage de suture selon la revendication 1, dans lequel le corps et la fixation de suture sont stériles et conditionnés dans une enceinte étanche aux bactéries.

3. Ancrage de suture selon la revendication 1, au moins une suture (44A-C) étant fixée à la fixation de suture.

4. Ancrage de suture selon la revendication 3, la ou les sutures comprenant une boucle de suture reliée sur la fixation de suture, la boucle ayant des extrémités (48) s'étendant vers l'extérieur à partir du corps d'ancrage de suture et de la fixation de suture.

5. Ancrage de suture selon la revendication 3, la ou les sutures comprenant une pluralité de boucles de suture (44A-C) de suture reliées à la fixation de suture, chaque boucle ayant des extrémités s'étendant vers l'extérieur à partir du corps d'ancrage de suture et de la fixation de suture.

6. Ancrage de suture selon la revendication 1, le corps comprenant une première surface de butée (36 ; 96) à l'intérieur de l'alésage axial, et la fixation de suture comprenant une seconde surface de butée opposée (88) en prise avec la première surface de butée de telle sorte que la fixation de suture peut tourner dans l'alésage axial contre la première surface de butée, et la mise en prise entre la première surface de butée et la seconde surface de butée empêche la fixation de suture de se déplacer de manière proximale hors de l'alésage axial.

7. Ancrage de suture selon la revendication 6, la première surface de butée et la seconde surface de butée étant annulaires.
